# EUROPÄISCHE PATENTSCHRIFT

(11) **EP 0 536 232 B1**
(45) Veröffentlichungstag und Bekanntmachung des Hinweises auf die Patenterteilung: **06.09.1995**
(21) Anmeldenummer: 91912093.1
(22) Anmeldetag: 21.06.1991
(51) Int. Cl.: A61M 15/00, A61J 1/00, A61M 13/00

(54) **VORRICHTUNG UND VERFAHREN ZUR PULVERDOSIERUNG**
PROCESS AND DEVICE FOR DOSING POWDERS
PROCEDE ET DISPOSITIF DE DOSAGE DE POUDRES

(30) Priorität: 28.06.1990 DE 4020571; 31.01.1991 DE 4102793
(43) Veröffentlichungstag der Anmeldung: 14.04.1993
(73) Patentinhaber: BOEHRINGER INGELHEIM INTERNATIONAL GmbH, 55218 Ingelheim am Rhein (DE); BOEHRINGER INGELHEIM KG, 55216 Ingelheim (DE)
(72) Erfinder: GUPTE, Arun, Rajaram, D-6507 Ingelheim (DE); HOCHRAINER, Dieter, D-6530 Bingen (DE); POSS, Gerhard, D-6905 Schriesheim (DE); WITTEKIND, Jürgen, D-6000 Frankfurt am Main 70 (DE); ZIERENBERG, Bernd, D-6530 Bingen (DE); KNECHT, Adolf, D-6500 Mainz (DE)
(86) Internationale Anmeldenummer: EP9101153
(87) Internationale Veröffentlichungsnummer: WO9200115

(56) Entgegenhaltungen:
- WO-A-90/13328
- DE-A- 2 837 040

## Beschreibung

Die Erfindung betrifft eine Vorrichtung und Verfahren zur Dosierung geringer Mengen feinteiliger Pulver.

Geringe Mengen feinteiliger, vorzugsweise mikronisierter Pulver werden vor allem zu therapeutischen Zwecken angewendet, hauptsächlich in Form von Aerosolen für die inhalative Behandlung von Atemwegserkrankungen wie Asthma.

Eine derartige Vorrichtung ist aus der DE-A-1792201 bekannt.

Pulver dieser Art werden in Mengen, die meist deutlich unter 50 mg liegen, mit der Atemluft der Lunge des Patienten zugeführt. Dabei hat sich herausgestellt, daß die Wirkstoffteilchen, damit sie tief in die Lunge gelangen, eine Größe unter 10 µm haben sollten. Das schließt allerdings die Verwendung auch etwas gröberer Teilchen in den Zubereitungen, vor allem für etwaige Hilfsstoffe, nicht aus. Werden Teilchen unterschiedlicher Größe verwendet, so ist ein deutlicher Größenunterschied gegebenenfalls sogar erwünscht oder doch nicht störend.

Für die Applikation feiner Pulver ohne Zuhilfenahme von Treibgasen in der Atemwegstherapie wurden vor allem zwei Methoden entwickelt.

Die eine verwendet Hartgelatinekapseln, die jeweils eine Wirkstoffdosis und gegebenenfalls zusätzlich Hilfsstoffe enthalten, die andere entnimmt mittels einer Meßkammer eine bestimmte Pulvermenge aus einem Vorratsbehälter und mischt sie der Atemluft zu. Geräte für beide Methoden sind in größerer Zahl beschrieben worden, vgl. beispielsweise DE-OS 23 46 914; EP-OS 166 294.

Die Erfindung bietet nun einen neuen Weg für die Applikation feiner Pulver. Bei ihr verbinden sich Einfachheit der Herstellung wirkstoffbeladener Träger in vorteilhaft mit Dosiergenauigkeit und der Möglichkeit, auf einfache Weise ein für die Inhalation geeignetes Aerosol zu erzeugen.

Erfindungsgemäß wird ein velours- oder samtartiges Material mit dem zu dosierenden Pulver beladen und die gewünschte Menge des Pulvers mit einem Gasstrahl, eventuell nach mechanischer Lockerung, vorzugsweise einem Luftstrahl geeigneter Stärke vom Träger heruntergeblasen. Bei der inhalativen Anwendung wird das freigesetzte Pulver der Atemluft beigemischt, gewünschtenfalls unter Zwischenschaltung eines Inhalationsgefäßes, wie es z.B. in dem deutschen Gebrauchsmuster 89 08273 beschrieben ist.

Die im Prioritätsintervall der vorliegenden Anmeldung eingereichte PCT-Anmeldung WO 90/13328 schlägt Längliche Träger vor, die mit inhalierfähigem Pulver beladen sind. Träger mit velours- oder samtartiger Struktur sind jedoch nicht erwähnt.

Beim Einstreichen des trockenen Pulvers in das Veloursmaterial tritt durch die Bewegung der Fasern unter der Rakel eine Prädesagglomeration auf. Die Partikel befinden sich anschließend in einem sehr lockeren Zustand und neigen entsprechend wenig zum Verklumpen. Bei Aufbringen des Pulvers aus einer Suspension wird diese Prädesagglomeration durch Darüberstreichen mit einer Kante erreicht, so daß auch in diesem Falle nur ein Teil der Energie für die Desagglomeration vom Gasstrahl aufgebracht werden muß. Außerdem weisen die prädesagglomerierten Partikeln eine große Angriffsfläche für den Gasstrahl auf, was die Dispergierung ebenfalls günstig beeinflußt.

Der Träger besteht aus einem im wesentlichen ebenen Material, auf dem dünne Fasern angeordnet sind. Die Fasern sind mit einem Ende oder in der Mitte auf oder in dem Träger fixiert, wobei das freie Ende bzw. die beiden freien Enden nach oben gerichtet sind und Träger und Faser überwiegend Winkel von 45 - 90°, insbesondere 60 bis 90° miteinander bilden. Das Material, das die Fasern trägt, kann z.B. Papier, Kunststoff-Folie oder ein Gewebe sein; bei den Fasern kann es sich um natürliches oder synthetisches Material handeln, etwa um Baumwolle, Wolle, Seide, Viskose, Perlon, Nylon, Polyacryl.

Die Fasern haben eine Länge bis etwa 3 mm, vorzugsweise bis etwa 1 mm. Sie sollen nicht zu stark verfilzt sein, damit das aufgetragene Pulver, das im wesentlichen zwischen die Fasern eingelagert wird, relativ leicht wieder herausgeblasen werden kann. Die untere Grenze der Faserlänge liegt bei etwa 0,1 mm. Generell ist die Faserlänge so zu wählen, daß das aufgetragene Pulver in der gewünschten Menge pro Flächeneinheit untergebracht werden kann.

Dementsprechend ist für relativ große Pulvermengen pro Flächeneinheit ein Trägermaterial mit längeren Fasern zweckmäßig, während bei sehr kleinen Pulvermengen pro Flächeneinheit auch kürzere Fasern brauchbar oder sogar vorteilhaft sind. Die Menge des Pulvers, die auf eine Flächeneinheit aufgebracht werden kann, ist - außer von dem Trägermaterial - hauptsächlich von der Art (Dichte) des Pulvers und seiner Verdichtung abhängig. Wenn das Pulver inhalativ angewendet werden soll, muß jedoch darauf geachtet werden, daß nicht durch zu starkes Zusammenpressen die Dispergierung des Pulvers in dem angewendeten Gas- oder Luftstrahl beeinträchtigt wird.

Die Zahl der Fasern pro Flächeneinheit kann stark variiert werden. Verschiedene handelsübliche Träger haben sich als gut geeignet erwiesen (Velourfolie, Samt, Nicki). Diese Produkte liefern auch Anhaltspunkte für die geeignete Faserdichte bei anderen Trägern. Auch die Faserstärke kann innerhalb weiter Grenzen gewählt werden. Im allgemeinen werden Fasern mit einem Durchmesser von 0,002 bis 0,05, vorzugsweise 0,004 bis 0,03 mm verwendet. Der eigentliche samtartige Träger kann auch mit einer steifen Schicht verbunden, z.B. verklebt werden. Auch die Unterlegung mit einer saugfähigen Schicht kommt in Betracht.

Der Träger kann dementsprechend biegsam oder starr und z.B. rechteckig oder kreisförmig sein. Bevorzugt hat der Träger die Form eines Bandes. Dieses kann auf seiner gesamten Fläche oder auf einzelnen Teilflächen mit dem Pulver beladen sein. In letzterem Fall kann die Beladung des Bandes mittels einer Schablone auf einzelnen kleinen Flächen erfolgen, beispielsweise in Form kreisförmiger Flächen von wenigen Millimeter Durchmesser, die deutlichen Abstand voneinander haben, so daß beim Herausblasen des Pulvers von einer dieser Flächen das Pulver auf den benachbarten Flächen unberührt bleibt.

Mit der Menge des Pulvers auf jeder der kleinen beladenen Flächen kann somit die Dosis genau festgelegt werden.

Ist die gesamte Fläche des Trägers mit Pulver beschichtet, kann die Menge davon, die jeweils herausgeblasen wird, einfach durch die Größe der Fläche festgelegt werden, die bei jedem einzelnen Entnahmevorgang dem Gasstrahl ausgesetzt wird und die beispielsweise durch eine Maske begrenzt sein kann.

Notwendig ist eine Maske jedoch nicht. Es hat sich nämlich gezeigt, daß die durch den Gasstrahl vom gleichmäßig beladenen Träger heruntergeblasene Pulvermenge weitgehend konstant ist. Deshalb kann die dispergierte Pulvermenge auch durch die Stärke des Gasstrahls und die Düsengeometrie geregelt werden.

Zum Schutz der Pulverschicht kann es zweckmäßig sein, den Träger z.B. mit einer Kunststoff-Folie abzudecken bzw. so zu kaschieren, daß jeweils nur der Teil des Trägers offenliegt, von dem das Pulver entfernt werden soll. Insbesondere bei feuchtigkeitsempfindlichen Pulvern kommt auch eine beidseitige Aluminiumkaschierung in Betracht. Schließlich können auch Träger, z.B. Bänder, verwendet werden, bei denen mit Fasern besetzte Felder und glatte Felder abwechseln.

Zur Beladung des Trägers wird auf diesen zunächst eine 1 - 2 mm hohe Pulverschicht möglichst gleichmäßig aufgebracht (bei hochwirksamen Arzneimittelpulvern und auch bei Trägern mit sehr kurzen Fasern kann die Schicht auch erheblich dünner sein). Das Pulver wird mit einer Rakel in das Band gedrückt und überschüssiges Pulver abgestreift. Dieser Vorgang wird gewünschtenfalls ein- oder mehrmals wiederholt, wobei die Rakel schrittweise tiefer gestellt wird. Durch die Bewegung der Fasern unter dem Druck der Rakel erfolgt eine Desagglomeration der zusammengeballten Anteile des Pulvers.

Sollen nur bestimmte Stellen des Trägers beladen werden, kann der Träger beispielsweise mit einer Schablone, etwa einer entsprechend gelochten Folie, bedeckt werden. Wird nun das Pulver aufgebracht wie oben beschrieben, erfolgt eine Beladung nur an denjenigen Stellen, an denen sich die Löcher befinden.

Das Pulver kann auch in Form einer Suspension auf den Träger aufgebracht werden. Bei Arzneimitteln für die Inhalation ist die Wirkstoffdosis in der Regel so gering, daß die in einem Tropfen Suspension enthaltene Menge des Wirkstoffs ausreicht. Man bringt dann jeweils einen Tropfen der Suspension im gewünschten Abstand von dem nächsten Tropfen auf. Der Abstand wird so gewählt, daß der Pulverfleck, der nach dem Verdampfen des Suspensionsmittels zurückbleibt, deutlich von dem benachbarten Fleck abgegrenzt ist. Ziel ist es, bei der Überführung des Wirkstoffpulvers in den Strom der Atemluft jeweils nur genau die mit einem Tropfen aufgebrachte Pulvermenge vom Träger zu trennen.

Die Beladung des Trägers mit einer genau dosierten Pulvermenge auf einer kleinen Fläche ist mit Hilfe einer Suspension besonders gut zu bewerkstelligen.

Als Suspensionsmittel dienen flüssige organische Verbindungen, in denen das aufzutragende Pulver schwer löslich ist und die sich möglichst gut entfernen lassen.

Solche Suspensionsmittel, die in Abhängigkeit von den Löslichkeitseigenschaften des zu suspendierenden Stoffs bzw. Stoffgemischs ausgewählt werden, sind beispielsweise Dichlormethan, Essigester, 1,1,1-Trichlorethan oder Benzin (z.B. die Fraktion 60/95 oder 80/110). In der Regel werden der Suspension Suspendierhilfsstoffe zugesetzt, z.B. Lecithin. Der Feststoffgehalt in der Suspension beträgt meist zwischen 3 und 30 Gewichtsprozent, bevorzugt 5 bis 25 Gewichtsprozent; die Menge an Suspendierhilfsstoffe bewegt sich zwischen etwa 0,5 und 3 Gewichtsprozent, bezogen auf den Feststoff.

Träger und Suspension sollten so beschaffen sein, daß die Partikel des Pulvers an der Stelle zurückbleiben, wo der Tropfen in den Träger eindringt, während das Suspensionsmittel sich weiter verteilt und dann verdampft. Die Verdampfung kann durch Druckverminderung und/oder Wärme gefördert werden.

Das Material aus dem eingetrockneten Suspensionstropfen läßt sich im allgemeinen nicht ohne weiteres durch einen Luftstoß freisetzen und dispergieren. Werden aber die Fasern des Trägers bewegt, z.B. durch Darüberstreichen mit einer Kante, so werden die Verbindungen der Pulverpartikel wieder aufgebrochen und das Pulver wird "aktiviert". Die Einzelpartikel, die nach dem Aktivieren mit nur geringen Haftkräften aneinander oder an den Fasern des Trägers haften, können dann mit einem Luftstoß freigesetzt und zu einem hohen Anteil lungengängig dispergiert werden.

Statt das gelockerte Pulver durch einen Luftstrahl aus dem Träger herauszulösen, kann man es auch, unmittelbar bevor bzw. während der Strom der Atemluft am Träger vorbeigeleitet wird, durch Darüberstreifen mit einer Kante oder durch Ausbürsten lockern und in die eingeatmete Luft überführen.

Die Aufbringung eines Suspensionstropfens hat gegenüber der Aufbringung von trockenem Pulver außer der hohen Dosiergenauigkeit noch den Vorteil, daß das Pulver durch das Verkrusten zunächst gegen Freisetzung durch Beschleunigungen (Schlag, Vibrationen) geschützt ist. Die Aktivierung durch Streifen an einer Kante sollte dann erst unmittelbar vor der Freisetzung zur Inhalation erfolgen.

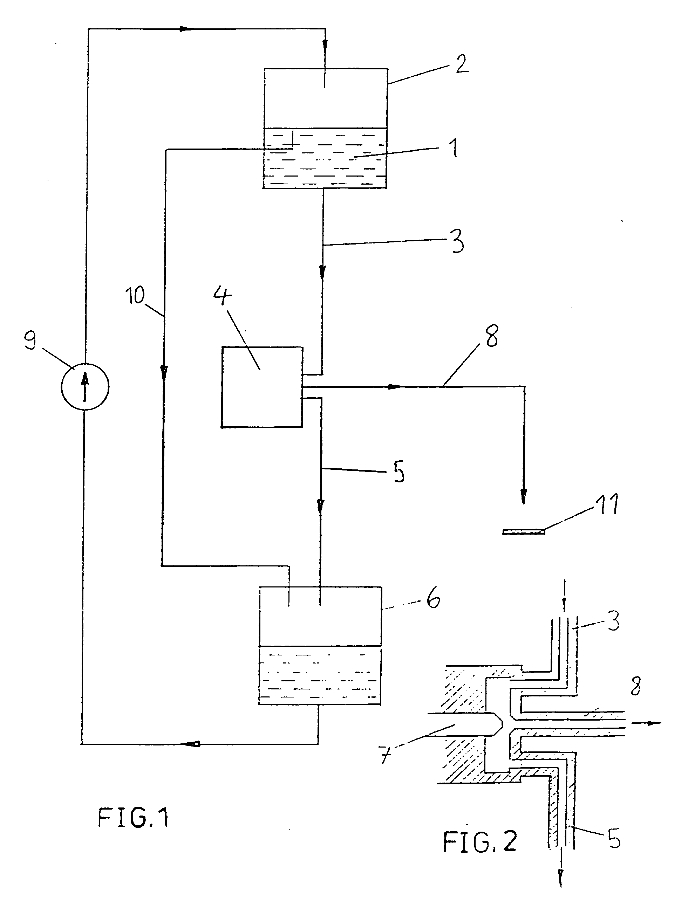

Eine Apparatur zum Auftragen der Suspensionstropfen gemäß der Erfindung ist in Figur 1 schematisch dargestellt. Die Suspension 1 befindet sich zunächst im Vorratsgefäß 2. Von dort fließt sie durch die Leitung 3 zu einem Magnetventil 4 und an den Ventilflächen vorbei durch die Leitung 5 in das Vorratsgefäß 6. Der Kolben 7 verschließt in Ruhestellung die Eintrittsöffnung der Leitung 8, aus der die Suspension auf den Träger 11 aufgebracht wird. Seitlich kann die Suspension am Kolben vorbei von der Leitung 3 zur Leitung 5 fließen. Wenn der Kolben durch einen Elektromagneten zurückgezogen wird, gibt er die Öffnung frei, so daß die Suspension auf den Träger 11 gelangen kann. Die Suspension aus dem Gefäß 6 wird mit der Pumpe 9 in das Vorratsgefäß 2 gepumpt. Damit die Suspension im Vorratsgefäß 2 stets gleiches Niveau hat, befindet sich zwischen diesen beiden Vorratsgefäßen eine Verbindungsleitung 10, durch die Suspension vom Vorratsgefäß 2 ins Vorratsgefäß 6 fließen kannn, wenn der Flüssigkeitsspiegel die Eintrittsöffnung dieser Verbindungsleitung übersteigt. Der Querschnitt der Leitungen 3 und 10 ist so klein gewählt, daß der Volumenstrom der durch die beiden Leitungen abfließenden Suspension geringer ist, als der Volumenstrom, den die Pumpe 9 fördert. In den Vorratsgefäßen 2 und 6 befinden sich Rührer, die die Partikel ständig in Schwebe halten. Zur gleichmäßigen Dosierung wird das Magnetventil 4 von einem elektronischen Taktgeber gesteuert. Einzelheiten des Ventils sind in Figur 2 dargestellt.

### Beispiel für das Auftragen des Arzneimittels als Suspension:

Eine Suspension von mikronisiertem Fenoterol (Anteil an Fenoterol: (10 Gewichtsprozent) in Dichlormethan unter Zusatz von (0,1 Gewichtsprozent) Lecithin wurde auf Samt mit einer Faserlänge von 1,2 mm über Grundgewebe tropfenweise im Abstand von ca. 10 mm aufgetragen und getrocknet. Das Pulver wurde dann durch Darüberstreifen mit einer Kante aktiviert und mit einem leichten Druckluftstoß aus dem Träger herausgeblasen.
Der inhalierbare Anteil der ausgebrachten Partikel (Partikeldurchmesser < 5,8 µm) lag bei 41,4 % der ausgebrachten Dosis.

Für die Dispergierung (das Herausblasen) des Pulvers genügt eine relativ geringe Gasmenge, z.B. 10 cm³ Luft, die durch eine Düse von 0,5 mm Durchmesser gedrückt wird.

Der zum Dispergieren benötigte Gastrahl kann auf verschiedene Weise erzeugt werden, etwa mittels eines mit einer Düse versehenen Zylinders, aus dem Luft durch einen federbetriebenen Kolben herausgedrückt wird, oder mittels üblicher, zur Druckerzeugung verwendbarer kleiner CO₂-Behälter. Statt des Zylinders mit Kolben kann auch ein Faltenbalg verwendet werden.

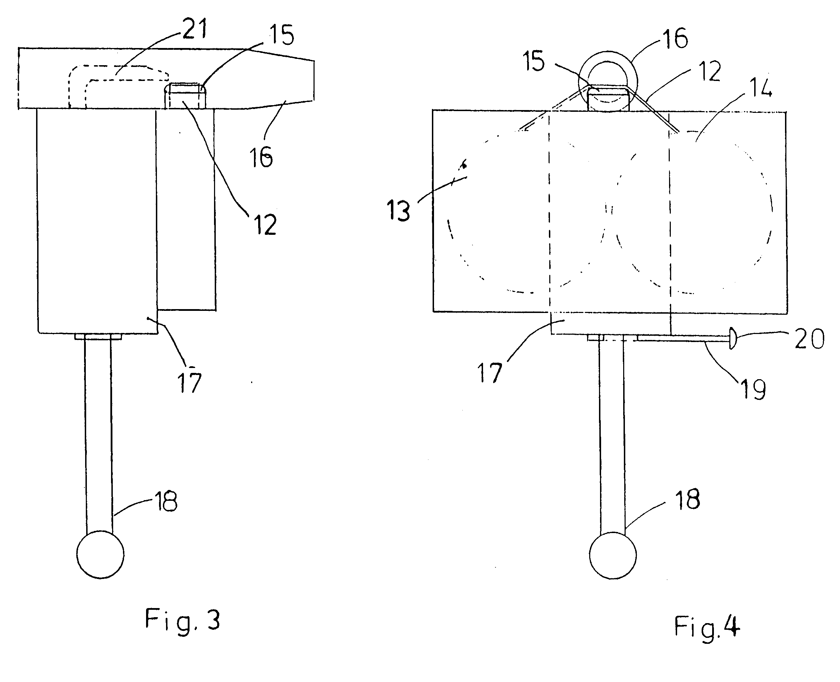

Ein einfaches Gerät, in dem erfindungsgemäß beladene Träger angewendet werden können, ist in den Figuren 3 und 4 schematisch dargestellt. Hauptbestandteile sind zwei Spulen, von denen die eine das beladene, die andere das verbrauchte Band aufnimmt. Das Band wird über eine Platte (Tisch) geführt, wobei es an einer Kante zur Aktivierung des Pulvers vorbeigeführt wird. Hier trifft der Gas- bzw. Luftstrahl auf das Band und reißt das Pulver mit. Der Strahl wird im allgemeinen zu einem Zeitpunkt ausgelöst, wo die Atemluft durch das Mundrohr strömt. Zweckmäßig ist es, den Gas- bzw. Luftstrahl durch die Atemluft auszulösen, um dadurch die Dispergierung des Pulvers mit dem Inhalationsvorgang zu koordinieren.

In Figur 3 ist ein Inhalationsgerät von vorn dargestellt. Das Trägerband 12 mit dem Pulver, das in einzelnen Flecken in regelmäßigem Abstand auf das Band aufgebracht ist, wird von der Spule 13 abgewickelt und, nach Entleerung, auf Spule 14 aufgewickelt. Durch einen in üblicher Weise konstruierten Transportmechanismus wird das Band jeweils bei Betätigung des Geräts um soviel weitergespult, daß ein neuer Fleck auf den Tisch 15 gelangt. Der Tisch 15 befindet sich in einem Mundrohr 16, durch dar der Patient einatmet. Der Schlitz, durch den das Trägerband 12 in das Mundrohr hineingeführt wird, ist etwas enger als das Trägerband 12 dick ist. Dabei ist die obere Begrenzung des Schlitzes als Kante zur Aktivierung des Pulvers ausgebildet. Aus einem Zylinder 17, in dem sich ein unter Federdruck stehender Kolben mit dem Handgriff 18 befindet, wird durch eine Düse Luft auf den Pulverfleck getrieben. Die Feder, die den Kolben in Richtung auf den Tisch 15 drückt, wird durch Zug an dem Handgriff 18 gespannt. Mittels einer Arretiervorrichtung 19, die durch Drücken auf den Knopf 20 gelöst werden kann, wird der Dispergierungsschritt vorbereitet. Während des Einatmens durch das Mundrohr 16 drückt der Patient auf den Knopf 20 und erreicht damit, daß der Kolben die im Zylinder enthaltene Luftmenge durch die Düse auf den Pulverfleck treibt, so daß das Pulver in der Atemluft dispergiert wird.

Figur 4 zeigt schematisch das Gerät von der Seite, wobei das Mundrohr 16 im Schnitt zu sehen ist. Die Düse 21 lenkt den Luftstrom aus dem Zylinder 17 auf den Pulverfleck.

Die Spulen mit dem Träger sind hier in einer Kassette, ähnlich den bei Kassettenrekordern üblichen, enthalten. Ihr Transport wird vorteilhaft mit der Bewegung des Handgriffs 18 gekoppelt, und zwar in der Weise, daß bei jedem Spannen des Kolbens das Trägerband so weiterbewegt wird, daß der nächste Fleck auf den Tisch 15 gelangt.

Statt des Zylinders mit Feder kann - wie erwähnt - z.B. zur Erzeugung eines Gasstroms auch ein Faltenbalg oder eine CO₂-Patrone üblicher Art verwendet werden, die bei jeder Anwendung des Geräts einige cm³ CO₂ abgibt. Das CO₂ wird dann gleichfalls durch eine Düse auf die mit Pulver beschichtete Trägerfläche gelenkt. Das Gasvolumen, das zur Dispergierung einer für therapeutische Zwecke erforderlichen Pulvermenge benötigt wird, beträgt in der Regel 3 - 20 cm³ (unter Normalbedingungen).

Je nach der Art des Trägers werden Düsen mit einer geeignet geformten Mündung verwendet. Im Fall der kreisrunden Pulverflecke hat die Düse ein kleine, vorzugsweise runde Mündung. Bei größeren pulverbeschichteten Tragerflächen ist gegebenenfalls eine schlitzförmige oder rechteckige Düse günstiger. In diesem Fall wird erforderlichenfalls eine größere Gasmenge angewendet, um die nötige Ausströmungsgeschwindigkeit zu gewährleisten.

In einer Versuchsreihe wurde die Genauigkeit der Dosierung ermittelt, wobei entweder (Versuch 1, 2, 3) ein Trägerband kontinuierlich durch Aufbringen trockenen Pulvers belegt war und jeweils ein scharf begrenztes Stück des Bandes untersucht wurde oder es wurde ein Band verwendet, bei dem nur bestimmte Stellen mit Pulver belegt waren.

Folgende Flächenbelegungen und relativen Standardabweichungen wurden ermittelt:

| Material | Flächenbelegung | relative Standardabweichung |
|---|---|---|
| 1. Veloursfolie | 6,6 mg/cm² | 6,3 % |
| 2. Samt | 2,2 mg/cm² | 6,0 % |
| 3. Nicki | 5,4 mg/cm² | 6,2 % |
| 4. Veloursfolie mit einzelnen kreisförmigen Pulverflecken* | 2,1 mg/Fleck | 11,2 % |

| | | |
|---|---|---|
| * Beim Auftragen des Pulvers war der Träger mit einer gelochten Folie abgedeckt. Die Löcher hatten einen Durchmesser von 4 mm und einen Abstand von 10 - 15 mm voneinander. Durch Verbesserung der Beladung konnte in weiteren Versuchen die Standardabweichung noch gesenkt werden. | | |

Für die praktisch vollständige Dispergierung beim Herausblasen des Pulvers genügt eine relativ geringe Gasmenge, z.B. 10 cm³ Luft, die durch eine Düse von 0,8 mm Durchmesser gedrückt wird (im Fall der Pulverflecken nach Versuch 4). Dabei ist eine hervorragende Desagglomeration festzustellen.

Wie mit Hilfe des Andersen-Impaktors unter Verwendung von mikronisiertem Fenoterol ermittelt wurde, lagen 40 % der Teilchen der ausgebrachten Dosis im Partikelgrößenbereich unter 5,8 µm.

## Patentansprüche

1. Mit pulverförmiger Arzneimittelzubereitung für die Erzeugung von Inhalationsaerosolen beladener Träger (11), dadurch gekennzeichnet, daß er aus einem im wesentlichen flachen Material besteht, auf dem Fasern samt- oder veloursartig angeordnet sind und das Pulver zwischen den Fasern eingelagert ist.

2. Pulverbeladener Träger nach Anspruch 1, dadurch gekennzeichnet, daß die Faserlänge 0,1 bis 3 mm beträgt.

3. Pulverbeladener Träger nach Anspruch 1 oder 2, dadurch gekennzeichnet, daß die die Fasern tragende Schicht flexibel ist.

4. Pulverbeladener Träger nach Anspruch 1 oder 2, dadurch gekennzeichnet, daß die die Fasern tragende Schicht starr ist.

5. Pulverbeladener Träger nach einem der Ansprüche 1 bis 4, dadurch gekennzeichnet, daß die gesamte Trägerfläche gleichmäßig mit Pulver beladen ist.

6. Pulverbeladener Träger nach einem der Ansprüche 1 bis 4, dadurch gekennzeichnet, daß nur Teilflächen des Trägers mit Pulver beladen sind, wobei die einzelnen beladenen Teilflächen des Trägers jeweils eine Einzeldosis eines Arzneistoffs enthalten.

7. Pulverbeladener Träger nach einem der Ansprüche 1 bis 6, dadurch gekennzeichnet, daß der Träger auf der pulverbeladenen Seite oder beidseitig mit einer Schutzfolie versehen ist.

8. Verfahren zur Dosierung von Pulvern für die Inhalation, dadurch gekennzeichnet, daß mittels eines Gasstrahls aus einem Träger nach einem der Ansprüche 1 bis 7 eine definierte Menge des Pulvers herausgeblasen und in der Atemluft dispergiert wird.

9. Verfahren nach Anspruch 8, dadurch gekennzeichnet, daß diejenige Fläche des Trägers, aus der das Pulver herausgeblasen werden soll, von einer Maske begrenzt wird.

10. Verfahren zur Herstellung eines Trägers nach einem der Ansprüche 1 bis 7, dadurch gekennzeichnet, daß das Pulver auf dem Träger gleichmäßig verteilt und mit einer Rakel in einer oder mehreren Stufen in die Faserschicht eingearbeitet wird.

11. Verfahren zur Herstellung eines Trägers nach einem der Ansprüche 1 bis 7, dadurch gekennzeichnet, daß das Pulver auf den samt- oder veloursartigen Träger in Form einer kleinen Menge einer Suspension aufgetragen und das Suspensionsmittel verdampft wird.

12. Verfahren zur Dosierung von Pulvern für die Inhalation, dadurch gekennzeichnet, daß Einzeldosen des Pulvers auf einen Träger nach einem der Ansprüche 1 bis 7 in Form einer Suspension aufgetragen, nach dem Trocknen aktiviert, mit Hilfe eines Gasstrahls vom Träger geblasen und in der Atemluft dispergiert werden.

## Claims

1. Carrier (11) charged with powdered pharmaceutical preparation for the production of aerosols for inhalation, characterised in that it consists of a substantially flat material on which fibres are arranged in a velvet- or velour-like manner and the powder is incorporated between the fibres.

2. Powder-charged carrier according to claim 1, characterised in that the fibre length is 0.1 to 3 mm.

3. Powder-charged carrier according to claim 1 or 2, characterised in that the layer which carries the fibres is flexible.

4. Powder-charged carrier according to claim 1 or 2, characterised in that the layer which carries the fibres is rigid.

5. Powder-charged carrier according to one of claims 1 to 4, characterised in that the entire carrier surface is uniformly charged with powder.

6. Powder-charged carrier according to one of claims 1 to 4, characterised in that only partial areas of the carrier are charged with powder, the individual charged partial areas of the carrier each containing a single dose of a pharmaceutical substance.

7. Powder-charged carrier according to one of claims 1 to 6, characterised in that the carrier is provided with a protective film on the powder-charged side or on both sides.

8. Process for metering powders for inhalation, characterised in that, by means of a gas jet, a defined amount of powder is blown out from a carrier according to one of claims 1 to 7 and dispersed in the air breathed in.

9. Process according to claim 8, characterised in that the area of the carrier from which the powder is to be blown out is defined by a mask.

10. Process for producing a carrier according to one of claims 1 to 7, characterised in that the powder is uniformly distributed on the carrier and is incorporated in the fibre layer in one or more steps using a doctor blade.

11. Process for producing a carrier according to one of claims 1 to 7, characterised in that the powder is applied to the velvet- or velour-like carrier in the form of a small amount of a suspension and the suspending agent is evaporated off.

12. Process for metering powders for inhalation, characterised in that single doses of the powder are applied to a carrier according to one of claims 1 to 7 in the form of a suspension, then activated after drying, blown out of the carrier by means of a gas jet and dispersed in the air breathed in.

## Revendications

1. Support (11) chargé d'une préparation médicamenteuse pulvérulente pour la production d'aérosols pour l'inhalation, caractérisé en ce qu'il consiste en un matériau sensiblement plat sur lequel des fibres sont disposées à la manière de la peluche ou du velours et la poudre est déposée entre les fibres.

2. Support chargé de poudre selon la revendication 1, caractérisé en ce que la longueur des fibres est de 0,1 à 3 mm.

3. Support chargé de poudre selon la revendication 1 ou 2, caractérisé en ce que la couche qui porte les fibres est flexible.

4. Support chargé de poudre selon la revendication 1 ou 2, caractérisé en ce que la couche qui porte les fibres est rigide.

5. Support chargé de poudre selon l'une des revendications 1 à 4, caractérisé en ce que toute la surface du support est chargée de poudre uniformément.

6. Support chargé de poudre selon l'une des revendications 1 à 4, caractérisé en ce que seules des surfaces partielles du support sont chargées de poudre, les différentes surfaces partielles chargées du support contenant chacune une dose unitaire d'un médicament.

7. Support chargé de poudre selon l'une des revendications 1 à 6, caractérisé en ce que le support est muni d'une feuille de protection sur le côté chargé de poudre ou des deux côtés.

8. Procédé de dosage de poudres pour l'inhalation, caractérisé en ce qu'au moyen d'un jet de gaz une quantité définie de poudre est expulsée d'un support selon l'une des revendications 1 à 7 et est dispersée dans l'air inhalé.

9. Procédé selon la revendication 8, caractérisé en ce que la surface du support de laquelle la poudre doit être expulsée est limitée par un masque.

10. Procédé de production d'un support selon l'une des revendications 1 à 7, caractérisé en ce que la poudre est répartie uniformément sur le support et est incorporée en une ou plusieurs étapes dans la couche de fibres avec une racle.

11. Procédé de production d'un support selon l'une des revendications 1 à 7, caractérisé en ce que la poudre est appliquée sur le support de type peluche ou velours sous forme d'une petite quantité d'une suspension et l'agent de suspension est évaporé.

12. Procédé de dosage de poudres pour l'inhalation, caractérisé en ce que des doses unitaires de la poudre sont appliquées sous forme d'une suspension sur un support selon l'une des revendications 1 à 7, activées après le séchage, expulsées du support à l'aide d'un jet de gaz et dispersées dans l'air inhalé.
